(19) Europäisches Patentamt
European Patent Office
Office européen des brevets

(11) EP 0 797 997 B1

(12) **EUROPÄISCHE PATENTSCHRIFT**

(45) Veröffentlichungstag und Bekanntmachung des Hinweises auf die Patenterteilung:
**15.06.2005 Patentblatt 2005/24**

(51) Int Cl.[7]: **A61K 38/08**, A61K 38/04, A61P 1/16

(21) Anmeldenummer: **97104345.0**

(22) Anmeldetag: **14.03.1997**

(54) **Verwendung von peptidischen Bradykinin-Antagonisten zur Herstellung von Arzneimitteln**

Use of peptidic bradykinin antagonists for the preparation of medicaments

Utilisation d'antagonistes peptidiques de bradykinine pour la préparation de médicaments

(84) Benannte Vertragsstaaten:
**AT BE CH DE DK ES FI FR GB GR IE IT LI LU NL PT SE**
Benannte Erstreckungsstaaten:
**SI**

(30) Priorität: **27.03.1996 DE 19612067**

(43) Veröffentlichungstag der Anmeldung:
**01.10.1997 Patentblatt 1997/40**

(73) Patentinhaber: **Aventis Pharma Deutschland GmbH**
**65929 Frankfurt am Main (DE)**

(72) Erfinder:
- **Breipohl, Gerhard, Dr.**
  **60529 Frankfurt (DE)**
- **Henke, Stephan, Dr.**
  **65719 Hofheim (DE)**
- **Knolle, Jochen Dr.**
  **65830 Kriftel (DE)**
- **Wirth Klausm Dr.**
  **65830 Kriftel (DE)**
- **Hropot, Max, Dr.**
  **65439 Flörsheim (DE)**
- **Bickel, Martin, Dr.**
  **61348 Bad Homburg (DE)**

(56) Entgegenhaltungen:
**EP-A- 0 370 453**
**EP-A- 0 552 106**
**EP-A- 0 622 361**

- **SHIBAYAMA Y.: "An experimental study into the cause of acute haemorrhagic gastritis in cirrhosis" JOURNAL OF PATHOLOGY, Bd. 149, Nr. 4, August 1986 (1986-08), Seiten 307-313, XP000882514**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 797 997 B1

Printed by Jouve, 75001 PARIS (FR)

## Beschreibung

Die Erfindung betrifft die Verwendung von peptidischen Bradykinin-Antagonisten zur Herstellung von Arzneimitteln zur Behandlung von chronisch fibrogenetischen Lebererkrankungen (Leberzirrhose und Leberfibrose) und akuten Lebererkrankungen und zur Prävention von Komplikationen.

Bradykinin und verwandte Peptide sind potente Entzündungen und Schmerzen erzeugende und vasoaktive körpereigene Substanzen. Die Verwendung von Bradykinin-Antagonisten als Mittel zur Bekämpfung von Zuständen, die durch Bradykinin vermittelt, ausgelöst oder unterstützt werden, ist bekannt (EP-B O 370 453).

EP-A-622 361 offenbart nicht-peptidische Bradykinin-Antagonisten zur Behandlung von entzündlichen Krankheiten.

EP-A-552 106 beschreibt peptidische Bradykinin-Antagonisten der Formel (4-Guanidinobenzoyl)-Arg-Pro-Hyp-Gly-Thia-Ser-Tic-Oic-Arg-OH zur Behandlung von unter anderem traumatischen Zuständen und Entzündungen.

Shibayama (Journal of Pathology 1986, 149(4), 307-313) beschreibt den Zusammenhang zwischen endotoxin-induziertem Bradykinin und der Erhöhung der Permeabilität der Venen bei zirrhotischen Patienten mit gastrointestinalen Blutungen.

Überraschenderweise wurde nun gefunden, daß peptidische Bradykinin-Antagonisten geeignete Mittel zur Behandlung von chronisch fibrogenetischen Lebererkrankungen (Leberzirrhose und Leberfibrose) und akuten Lebererkrankungen und zur Prävention von Komplikationen, insbesondere zur Prophylaxe bzw. Behandlung der portalen Hypertonie, Dekompensationserscheinungen wie Aszites, Ödembildung, hepatorenales Syndrom, hypertensive Gastro- und Colopathie, Splenomegalie sowie Blutungskomplikationen im Gastrointestinaltrakt durch portale Hypertonie, Kollateralkreislauf und Hyperämie und eine Kardiopathie als Folge einer chronisch hyperdynamen Kreislaufsituation und deren Folgen, sind.

Als Verbindungen eignen sich peptidische Bradykinin-Antagonisten, die im Modell der $CCl_4$-induzierten Leberfibrose an der Ratte einen natriuretischen Effekt zeigen und durch die Formel I gekennzeichnet sind

$$Z - P - A - B - C - E - F - K - (D)Q - G - M - F' - I \qquad (I),$$

in welcher bedeuten:

Z a$_1$) Wasserstoff, ($C_1$-$C_8$)-Alkyl, ($C_1$-$C_8$)-Alkanoyl, ($C_1$-$C_8$)-Alkoxycarbonyl, ($C_3$-$C_8$)-Cycloalkyl, ($C_4$-$C_9$)-Cycloalkanoyl, oder ($C_1$-$C_8$)-Alkylsulfonyl,

in welchen jeweils 1, 2 oder 3 Wasserstoffatome gegebenenfalls durch 1, 2 oder 3 gleiche oder verschiedene Reste aus der Reihe

Carboxy, NHR(1), [($C_1$-$C_4$)-Alkyl]NR(1) oder [($C_6$-$C_{10}$)-Aryl-($C_1$-$C_4$)-alkyl]NR(1), wobei R(1) für Wasserstoff oder eine Urethan-Schutzgruppe steht, ($C_1$-$C_4$)-Alkyl, ($C_1$-$C_8$)-Alkylamino, ($C_6$-$C_{10}$)-Aryl-($C_1$-$C_4$)-alkylamino, Hydroxy, ($C_1$-$C_4$)-Alkoxy, Halogen, Di-($C_1$-$C_8$)-alkylamino, Di-[($C_6$-$C_{10}$)-aryl-($C_1$-$C_4$)]-alkylamino, Carbamoyl, Phthalimido, 1,8-Naphthalimido, Sulfamoyl, ($C_1$-$C_4$)-Alkoxycarbonyl, ($C_6$-$C_{14}$)-Aryl und ($C_6$-$C_{14}$)-Aryl-($C_1$-$C_5$)-alkyl ersetzt sind,

oder in welchen jeweils 1 Wasserstoffatom gegebenenfalls durch einen Rest aus der Reihe

($C_3$-$C_8$)-Cycloalkyl, ($C_1$-$C_6$)-Alkylsulfonyl, ($C_1$-$C_6$)-Alkylsulfinyl, ($C_6$-$C_{14}$)-Aryl-($C_1$-$C_4$)-alkylsulfonyl, ($C_6$-$C_{14}$)-Aryl-($C_1$-$C_4$)-alkylsulfinyl, ($C_6$-$C_{14}$)-Aryl,

($C_6$-$C_{14}$)-Aryloxy, ($C_3$-$C_{13}$)-Heteroaryl und ($C_3$-$C_{13}$)-Heteroaryloxy

und 1 oder 2 Wasserstoffatome durch 1 oder 2 gleiche oder verschiedene Reste aus der Reihe

Carboxy, Amino, ($C_1$-$C_8$)-Alkylamino, Hydroxy, ($C_1$-$C_4$)-Alkoxy, Halogen, Di-($C_1$-$C_8$)-alkylamino, Carbamoyl, Sulfamoyl, ($C_1$-$C_4$)-Alkoxycarbonyl, ($C_6$-$C_{14}$)-Aryl und ($C_6$-$C_{14}$)-Aryl-($C_1$-$C_5$)-alkyl ersetzt sind;

a$_2$) ($C_6$-$C_{14}$)-Aryl, ($C_7$-$C_{15}$)-Aroyl, ($C_6$-$C_{14}$)-Arylsulfonyl, ($C_3$-$C_{13}$)-Heteroaryl, oder ($C_3$-$C_{13}$)-Heteroaroyl;

a$_3$) Carbamoyl, das gegebenenfalls am Stickstoff durch ($C_1$-$C_8$)-Alkyl, ($C_6$-$C_{14}$)-Aryl oder ($C_6$-$C_{14}$)-Aryl-($C_1$-$C_5$)-alkyl substituiert sein kann;

wobei in den unter a$_1$), a$_2$) und a$_3$) definierten Resten die Aryl-, Heteroaryl-, Aroyl-, Arylsulfonyl- und Heteroaroyl-Gruppen gegebenenfalls durch 1, 2, 3 oder 4 Reste aus der Reihe

Carboxy, Amino, Nitro, ($C_1$-$C_8$)-Alkylamino, Hydroxy, ($C_1$-$C_6$)-Alkyl, ($C_1$-$C_6$)-Alkoxy, ($C_6$-$C_{14}$)-Aryl, ($C_7$-$C_{15}$)-Aroyl, Halogen, Cyano, Di-($C_1$-$C_8$)-alkylamino, Carbamoyl, Sulfamoyl und ($C_1$-$C_6$)-Alkoxycarbonyl substituiert sind;

P eine direkte Bindung oder einen Rest der Formel II,

- NR(2) -(U)- CO - (II)

worin

R(2) Wasserstoff, Methyl oder eine Urethan-Schutzgruppe bedeutet,

U ($C_3$-$C_8$)-Cycloalkyliden, ($C_6$-$C_{14}$)-Aryliden, ($C_3$-$C_{13}$)-Heteroaryliden, ($C_6$-$C_{14}$)-Aryl-($C_1$-$C_6$)-alkyliden, welche gegebenenfalls substituiert sein können, oder $[CHR(3)]_n$ bedeutet,
wobei n 1 - 8, vorzugsweise 1 - 6 ist,

R(3) unabhängig voneinander Wasserstoff, ($C_1$-$C_6$)-Alkyl, ($C_3$-$C_8$)-Cycloalkyl, ($C_6$-$C_{14}$)-Aryl, ($C_3$-$C_{13}$)-Heteroaryl, bedeutet, die mit Ausnahme des Wasserstoffs jeweils gegebenenfalls durch Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, . Phthalimido, 1,8-Naphthalimido, 4-Imidazolyl, 3-Indolyl, 2-Thienyl, 3-Thienyl, 2-Pyridyl, 3-Pyridyl oder Cyclohexyl monosubstituiert sind,

wobei substituiertes Amino bevorzugt für -N(A')-Z, substituiertes Amidino bevorzugt für -(NH =)C-NH-Z, substituiertes Guanidino bevorzugt für -N(A')-C[=N(A')]-NH-Z und substituiertes Ureido bevorzugt für -CO-N(A') -Z stehen, in denen A' unabhängig voneinander Wasserstoff oder Z bedeutet, wobei Z wie unter $a_1$) oder $a_2$) definiert ist;
oder
worin R(2) und R(3) zusammen mit den diese tragenden Atomen ein mono-, bi- oder tricyclisches Ringsystem mit 2 bis 15 C-Atomen bilden;

A wie P definiert ist;

B eine basische Aminosäure in der L- oder D-Konfiguration, die in der Seitenkette substituiert sein kann;

C eine Verbindung der Formel III a oder III b

G'-G'-Gly

(III a)

G'-NH-$(CH_2)_p$-CO

(III b)

worin

p 2 bis 8 ist, und

G' unabhängig voneinander einen Rest der Formel IV

-NR(4)-CHR(5)-CO- (IV)

worin
R(4) und R(5) zusammen mit den diese tragenden Atomen ein heterocyclisches mono-, bi- oder tricyclisches Ringsystem mit 2 bis 15 C-Atomen bilden;

E den Rest einer neutralen, sauren oder basischen, aliphatischen oder alicyclisch-aliphatischen Aminosäure;

F unabhängig voneinander den Rest einer neutralen, sauren oder basischen, aliphatischen oder aromatischen Aminosäure, die in der Seitenkette substituiert sein kann, oder eine direkte Bindung;

(D)Q D-Tic, D-Phe, D-Oic, D-Thi oder D-Nal, die gegebenenfalls durch Halogen, Methyl oder Methoxy substituiert sein können oder einen Rest der nachstehenden Formel (V)

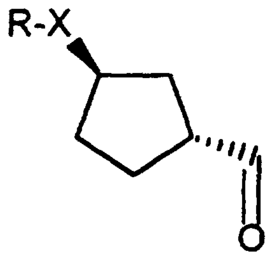

(V)

worin

X für Sauerstoff, Schwefel oder eine direkte Bindung steht;

R Wasserstoff, $(C_1-C_8)$-Alkyl, $(C_3-C_8)$-Cycloalkyl, $(C_6-C_{14})$-Aryl, $(C_6-C_{14})$-Aryl-$(C_1-C_4)$-alkyl, wobei der Alicyclus gegebenenfalls durch Halogen, Methyl oder Methoxy substituiert sein kann;

G wie G' oben definiert ist oder eine direkte Bindung;

F' wie F definiert ist, einen Rest $-NH-(CH_2)_q-$, mit q = 2 bis 8, oder, falls G keine direkte Bindung bedeutet, eine direkte Bindung;

I -OH, $-NH_2$ oder $NHC_2H_5$;

K den Rest $-NH-(CH_2)_x-CO-$ mit x = 1 - 4 oder eine direkte Bindung, und

M wie F definiert ist,

sowie deren physiologisch verträgliche Salze.

Geeignete Bradykinin-Antagonisten sind zum Beispiel beschrieben in den Patent-Anmeldungen WO 95/07294 [Scios Nova, Pseudopeptide], WO 94/08607 [Scios Nova, Pseudopeptide], WO 94/06453 [Stewart, aliphatic amino acid in 5-position], WO 93/11789 [Nova], EP-A 552 106 [Adir], EP-A 578 521 [Adir], WO 94/19372 [Scios Nova, Cyclopeptide], EP-A 370 453 [Hoechst], EP-A 472 220 [Syntex], WO 92/18155 [Nova], WO 92/18156 [Nova], WO 92/17201 [Cortech] und WO 94/11021 [Cortech; Bradykinin-Antagonisten der Formel $X(BKA)_n$, worin X ein Bindeglied, BKA die Peptidkette eines Bradykinin-Antagonisten und n eine ganze Zahl größer als 1 ist; Bradykinin-Antagonisten der Formel X(BKA); und Bradykinin-Antagonisten der Formel (Y)(X)(BKA) mit Y gleich einem Liganden, welcher ein Antagonist oder ein Agonist für einen Nicht-Bradykinin-Rezeptor ist].

Besonders geeignet sind Peptide der Formel I, in welcher bedeuten:

Z Wasserstoff oder wie oben unter $a_1$), $a_2$) oder $a_3$) definiert,
P eine Bindung oder ein Rest der Formel II

$$- NR(2) -(U)- CO - \qquad (II)$$

mit U gleich CHR(3) und
R(3) wie oben definiert,
R(2) gleich H oder $CH_3$,
A eine Bindung.

Insbesondere sind Verbindungen der Formel I bevorzugt, in der bedeuten:

Z Wasserstoff oder wie oben unter $a_1$), $a_2$) oder $a_3$) definiert,
P eine Bindung oder ein Rest der Formel II

- NR(2) -(U)- CO - (II)

mit U gleich CHR(3) und
R(3) unabhängig voneinander Wasserstoff, ($C_1$-$C_6$)-Alkyl, ($C_3$-$C_8$)-Cycloalkyl, ($C_6$-$C_{14}$)-Aryl, ($C_3$-$C_{13}$)-Heteroaryl, die mit Ausnahme des Wasserstoffs jeweils gegebenenfalls durch
Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z und substituiertes Guanidino bevorzugt für
-N(A')-C[=N(A')]-NH-Z stehen, in denen A' unabhängig voneinander Wasserstoff oder Z bedeutet, wobei Z wie unter $a_1$) oder $a_2$) definiert ist; oder
worin R(2) und R(3) zusammen mit den diese tragenden Atomen ein mono-, bi- oder tricyclisches Ringsystem mit 2 bis 15 C-Atomen bilden,
R(2) gleich H oder $CH_3$;
A eine Bindung;
(D)Q D-Tic.

Bevorzugt geeignet sind:

H-D-Arg-Arg-Pro-Hyp-Gly-Thi-Ser-D-Tic-Oic-Arg-OH (HOE 140)
para-Guanidobenzoyl-Arg-Pro-Hyp-Gly-Thi-Ser-D-Tic-Oic-Arg-OH
H-D-Arg-Arg-Pro-Hyp-Gly-Phe-Ser-D-HypE(transpropyl)-Oic-Arg-OH
H-D-Arg-Arg-Pro-Hyp-Gly-Cpg-Ser-D-Cpg-Cpg-Arg-OH
H-D-Arg-Arg-Pro-Pro-Gly-Thi-Ser-D-Tic-Oic-Arg-OH
H-Arg(Tos)-Pro-Hyp-Gly-Thi-Ser-D-Tic-Oic-Arg-OH
H-Arg(Tos)-Pro-Hyp-Gly-Phe-Ser-D-Tic-Oic-Arg-OH
H-D-Arg-Arg-Pro-Hyp-Gly-Phe-Ser-D-Tic-Oic-Arg-OH
Fmoc-D-Arg-Arg-Pro-Hyp-Gly-Thi-Ser-D-Tic-Oic-Arg-OH
Fmoc-Aoc-D-Arg-Arg-Pro-Hyp-Gly-Thi-Ser-D-Tic-Oic-Arg-OH
Fmoc-ε-Aminocaproyl-D-Arg-Arg-Pro-Hyp-Gly-Thi-Ser-D-Tic-Oic-Arg-OH
Benzoyl-D-Arg-Arg-Pro-Hyp-Gly-Thi-Ser-D-Tic-Oic-Arg-OH
Cyclohexylcarbonyl-D-Arg-Arg-Pro-Hyp-Gly-Thi-Ser-D-Tic-Oic-Arg-OH
Fmoc-Aeg(Fmoc)-D-Arg-Arg-Pro-Hyp-Gly-Thi-Ser-D-Tic-Oic-Arg-OH
Fmoc-Aeg(Fmoc)-Arg-Pro-Hyp-Gly-Thi-Ser-D-Tic-Oic-Arg-OH
Indol-3-yl-acetyl-D-Arg-Arg-Pro-Hyp-Gly-Thi-Ser-D-Tic-Oic-Arg-OH
Dibenzylacetyl-D-Arg-Arg-Pro-Hyp-Gly-Thi-Ser-D-Tic-Oic-Arg-OH

und deren physiologisch verträglichen Salze.

Besonders geeignet sind
H-D-Arg-Arg-Pro-Hyp-Gly-Thi-Ser-D-Tic-Oic-Arg-OH (HOE 140),
para-Guanidobenzoyl-Arg-Pro-Hyp-Gly-Thi-Ser-D-Tic-Oic-Arg-OH
und deren physiologisch verträglichen Salze.

Ganz besonders geeignet ist H-D-Arg-Arg-Pro-Hyp-Gly-Thi-Ser-D-Tic-Oic-Arg-OH (HOE 140) und dessen physiologisch verträglichen Salze.

Die Anwendung kann enteral, parenteral - wie z. B. subkutan, i. m. oder i. v. -, nasal, rektal oder per Inhalation erfolgen. Die Dosierung des Wirkstoffs hängt vom Körpergewicht, Alter und von der Applikationsart ab.

Die pharmazeutischen Präparate der vorliegenden Erfindung werden in an sich bekannten Lösungs-, Misch-, Granulier- , Tabletten- oder Dragierverfahren hergestellt.

Zur parenteralen Applikation werden die aktiven Verbindungen oder deren physiologisch verträgliche Salze, gewünschtenfalls mit den pharmazeutisch üblichen Hilfsstoffen, beispielsweise zur Isotonisierung oder pH-Einstellung sowie Lösungsvermittler, Emulgatoren, oder anderen Hilfsstoffen, in Lösung, Suspension oder Emulsion gebracht.

Für die beschriebenen Arzneistoffe ist auch der Einsatz von injizierbaren Retardzubereitungen zur subkutanen oder intramuskulären Anwendung sinnvoll. Als Arzneiformen können z. B. ölige Kristallsuspensionen, Mikrokapseln, Mikropartikel, Nanopartikel oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren aufgebaut sein können. Andere denkbare Polymere sind Polyamide, Polyester, Polyacetate oder Polysaccharide.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung von festen Arzneiformen sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Auch orale Retardzubereitungen oder Zubereitungen mit magensaftresistenten Überzügen sind denkbar. Retardzubereitungen können auf der Basis von Fett-, Wachs- oder Polymereinbettungen aufgebaut sein. Möglich sind hierbei auch Mehrschicht- oder Manteltabletten oder Pellets.

Für die beschriebenen Arzneistoffe ist auch eine Applikation auf Schleimhäute zur Erzielung systemisch wirksamer Spiegel sinnvoll. Dies betrifft die Möglichkeit der intranasalen, inhalativen und rektalen Anwendung.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie Pulver, wäßrige, alkoholische oder ölige Suspensionen oder wäßrige, alkoholische oder ölige Lösungen. Wäßrigen intranasalen Zubereitungen können Chelatbildner, wie Ethylendiamin-N,N,N',N'-tetraessigsäure und Puffer wie Essigsäure, Phosphorsäure, Citronensäure, Weinsäure und deren Salze zugefügt werden. Mehrfachdosenbehälter enthalten Konservierungsmittel wie Benzalkoniumchlorid, Chlorbutanol, Chlorhexidin, Sorbinsäure, Benzoesäure, PHB-Ester oder Organoquecksilberverbindungen.

Die Applikation der Nasallösungen kann mittels Dosierzerstäuber erfolgen oder als Nasaltropfen mit viskositätserhöhendem Anteil bzw. Nasengels oder Nasencremes.

Für die inhalative Anwendung können Vernebler oder Druckgas-Packungen unter Verwendung inerter Trägergase benutzt werden.

Zur Applikation von Pulvern zur nasalen oder pulmonalen Inhalation sind spezielle Applikatoren erforderlich.

Die wirksame Dosis beträgt mindestens 0.001 mg/kg/Tag, vorzugsweise mindestens 0.01 mg/kg/Tag, höchstens 3 mg/kg/Tag, vorzugsweise 0.03 bis 1 mg//kg/Tag Körpergewicht in Abhängigkeit vom Schweregrad der Symptomatik, bezogen auf einen Erwachsenen von 75 kg Körpergewicht.

Die für Aminosäuren verwendeten Abkürzungen entsprechen dem in der Peptidchemie üblichen drei Buchstaben-Code wie er in Europ. J. Biochem 138,9 (1984) beschrieben ist. Weitere verwendete Abkürzungen sind nachfolgend aufgelistet:

| | |
|---|---|
| Aeg | N-(2-Aminoethyl)glycin |
| Cpg | Cyclopentylglycyl |
| Fmoc | 9-Fluorenylmethyloxycarbonyl |
| Nal | 2-Naphthylalanyl |
| Oic | cis,endo-Octahydroindol-2-carbonyl |
| Thi | 2-Thienylalanyl |
| Tic | 1,2,3,4-Tetrahydroisochinolin-3-ylcarbonyl |

Beispiel 1:

Wirkung von HOE 140 auf die Urin- und Elektrolytausscheidung an Ratten mit Tetrachlorkohlenstoff-induzierter Leberfibrose

Methode:

Verwendet wurden Wistar Ratten (Züchter Hoechst AG, Kastengrund) mit einem anfänglichen Körpergewicht von 120-150 g.

Auslösung der Leberfibrose:

Eine Leberfibrose wurde induziert wie von Bickel et al., J. Hepatol., 13 (Suppl. 3) (1991) 26-33 beschrieben. Die Tiere erhielten zweimal pro Woche Tetrachlorkohlenstoff ($CCl_4$) in einer Dosis von 1 ml/kg per os für mindestens

6 Wochen. Die Fibrose der Leber wurde über den Kollagengehalt der Leber und Leber-relevante Serumparameter (Bilirubin, ALAT, Gallensäuren) verifiziert.

Im Verlauf der Fibrogenase wurden die Tiere unter Standardbedingungen wie folgt gehalten: Tages-Nacht-Rhythmus (Hellphase von 6.30 bis 18.30), Raumtemperatur 22 ± 2 C° und relative Luftfeuchtigkeit 60 ± 10%. Die Tiere erhielten standardisiertes Rattenfutter (Altromin$^R$ 1321) und Wasser ad libitum.

Salurese- und Diureseversuch:

Zum Zeitpunkt des Diureseversuches hatten die Tiere ein Gewicht zwischen 200 und 320 g erreicht. Futter wurde bereits 16 h vor dem Versuch entzogen und während des gesamten Versuchs vorenthalten. Der freie Zugang zu Wasser war den Tieren noch bis zum eigentlichen Versuchsbeginn gestattet.
Die Tiere wurden für die Dauer des Diureseversuches in speziellen Diuresekäfigen gehalten. Eine kontrollierte Diurese wurde mit einer orale Gabe von 20 ml Wasser per kg Körpergewicht zum Zeitpunkt 0 h ausgelöst. Die Ausscheidung von Elektrolyten und Urinvolumina wurde in den Sammelperioden von 0-5 und 6-24 h getrennt für jedes Tier ermittelt. Fünf Tage später wurde der Versuch an den gleichen Tieren erneut durchgeführt unter Gabe von Bradykinin-Antagonisten. Tiere erhielten je 0.3 mg/kg HOE 140 s.c. zum Zeitpunkt 0 und 6 h, gelöst in 5 ml Kochsalzlösung pro kg Köpergewicht.

Natrium und Kalium wurden flammenphotometrisch bestimmt (Flammenphotometer Eppendorf, Hamburg). Chlorid wurde argentometrisch über potentiometrische Endpunktbestimmung gemessen (Chloridmeter Eppendorf, Hamburg). Die analytischen Ergebnisse wurden zur Berechnung der Urinausscheidung (ml/kg Körpergewicht) und Elektrolytausscheidung (mmol/lkg Körpergewicht) herangezogen.

Ergebnis:

Tabelle 1

| (Mittelwerte (MW) ± SD, n = 10) | | | | | |
|---|---|---|---|---|---|
| | | Sammelperiode 1-5 h | | Sammelperiode 6-24 h | |
| | | Kontrolle | HOE 140 | Kontrolle | HOE 140 |
| Urinvolumen | MW | 19,06 | 26,59* | 23,29 | 31,03* |
| (ml/kg) | SD | 5,69 | 4,82 | 8,57 | 11,81 |
| Natrium | MW | 0,21 | 0,48* | 1,43 | 4,10*** |
| (mmol/kg) | SD | 0,16 | 0,19 | 0,90 | 1,40 |
| Kalium | MW | 0,43 | 0,51 | 2,85 | 2,10* |
| (mmol/kg) | SD | 0,27 | 0,32 | 0,85 | 1,00 |
| Chlorid | MW | 0,31 | 0,35 | 0,87 | 3,27*** |
| (mmol/kg) | SD | 0,21 | 0,26 | 0,35 | 1,22 |

* $p < 0.05$;
** $p < 0.01$;
*** $p < 0.001$

Statistik:

Die Ergebnisse sind angegeben als arithmetische Mittel und Standardabweichung (SD). Die statistische Überprüfung erfolgte mit dem T-Test oder bei Abweichung von der Normalverteilung mit dem nicht-parametrischen Test nach Mann-Whitney.

Ergebnisse und Bewertung:

Mit Bradykinin-Antagonisten behandelte Tiere zeigen eine ausgeprägte Erhöhung der Natriumausscheidung an Ratten mit Tetrachlorkohlenstoff-induzierter Leberfibrose. Als Beispiel sind in Tabelle 1 experimentelle Daten mit dem peptidischen Bradykinin-Antagonisten Hoe 140 (INN Icatibant) aufgeführt. Es ergibt sich eine deutliche, statistisch signifikante Natriurese.

Das Modell der Tetrachlorkohlenstoff-induzierten Leberfibrose an der Ratte findet als Modell für die Leberzirrhose am Menschen allgemeine Anerkennung. Überschießende Natriumretention ist charakteristisch für die Leberfibrose und Leberzirrhose bei Mensch und Tier und wird als Folge einer tiefgreifenden hämodynamischen Störung betrachtet (Schrier et al., Hepatology 8 (1988) 1151-1157). Diese hämodynamische Störung besteht in einer portalen Hypertonie (Pfortaderhochdruck), eng verknüpft mit einer überschießenden peripheren Vasodilatation vor allem im Splanchnicusgebiet (hyperdyname Kreislaufsituation). Die Ursache der peripheren Vasodilatation war bisher nicht geklärt. Die pathologische Natrium- und Wasserretention verschlechtert ihrerseits die Symptomatik, indem sie z.B. zu Ödembildung und Aszites beiträgt. Die portale Hypertonie ist assoziiert mit inadäquater peripherer Vasodilatation und Natriumretention. Diese werden für Dekompensationserscheinungen bei Leberfibrose und Leberzirrhose verantwortlich gemacht. Diese Dekompensationserscheinungen umfassen nicht nur Symptome wie Ödembildung und Aszites, sondern auch das sogenannte hepatorenale Syndrom (Nierenversagen als Folge einer schweren Lebererkrankung).

Die starke natriuretische Wirkung von Bradykinin-Antagonisten bei Ratten mit Leberfibrose und Leberzirrhose ist unerwartet, weil Bradykinin-Antagonisten an gesunden Tieren diese Wirkung nicht zeigen und, im Gegenteil, in besonderen Hypertonie-Modellen sogar zu einer Verminderung der Diurese und Natriumausscheidung führen kann (Madeddu et al., Br. J. Pharmacol. 106 (1992) 380-386; Majima et al., Hypertension, 22 (1993) 705-714). Bradykinin kann nämlich in der Niere Salurese und Diurese über vaskuläre und tubuläre Mechanismen stimulieren.

Bradykinin ist ein endogenes Peptid mit stark vasodilatierenden und gefäßpermeabilitätssteigernden Eigenschaften in verschiedenen Gefäßgebieten.

Unsere Ergebnisse zeigen, daß Bradykinin ein wesentlicher Mediator der exzessiven Natriumretention ist. Eine verbesserte hämodynamische und mikrovaskuläre Situation überkompensiert bei weitem eine mögliche Einschränkung der Natrium- und Wasserausscheidung durch Hemmung der stimulierenden Wirkung endogenen Bradykinins in der Niere, so daß ein therapeutischer Nutzen resultiert.

Somit sind peptidische Bradykinin-Antagonisten geeignet für die therapeutische und präventive Behandlung bei chronischen fibrogenetischen Lebererkrankungen (Leberzirrhose und Leberfibrose) und akuten Lebererkrankungen und zur Prävention insbesondere des hepatorenalen Syndroms.

## Patentansprüche

**1.** Verwendung eines Bradykinin-Antagonisten der Formel I

$$Z - P - A - B - C - E - F - K - (D)Q - G - M - F' - I \qquad (I),$$

in welcher bedeuten:

Z a$_1$) Wasserstoff, $(C_1-C_8)$-Alkyl, $(C_1-C_8)$-Alkanoyl, $(C_1-C_8)$-Alkoxycarbonyl, $(C_3-C_8)$-Cycloalkyl, $(C_4-C_9)$-Cycloalkanoyl, oder $(C_1-C_8)$-Alkylsulfonyl,

in welchen jeweils 1, 2 oder 3 Wasserstoffatome gegebenenfalls durch 1, 2 oder 3 gleiche oder verschiedene Reste aus der Reihe

Carboxy, NHR(1), $[(C_1-C_4)$-Alkyl]NR(1) oder $[(C_6-C_{10})$-Aryl-$(C_1-C_4)$-alkyl]NR(1), wobei R (1) für Wasserstoff oder eine Urethan-Schutzgruppe steht, $(C_1-C_4)$-Alkyl, $(C_1-C_8)$-Alkylamino, $(C_6-C_{10})$-Aryl-$(C_1-C_4)$-alkylamino, Hydroxy, $(C_1-C_4)$-Alkoxy, Halogen,

Di-$(C_1-C_8)$-alkylamino, Di-$[(C_6-C_{10})$-aryl-$(C_1-C_4)$-alkyl]amino, Carbamoyl, Phthalimido, 1,8-Naphthalimido, Sulfamoyl, $(C_1-C_4)$-Alkoxycarbonyl, $(C_6-C_{14})$-Aryl und $(C_6-C_{14})$-Aryl-$(C_1-C_5)$-alkyl ersetzt sind,

oder in welchen jeweils 1 Wasserstoffatom gegebenenfalls durch einen Rest aus der Reihe

$(C_3-C_8)$-Cycloalkyl, $(C_1-C_6)$-Alkylsulfonyl,
$(C_1-C_6)$-Alkylsulfinyl, $(C_6-C_{14})$-Aryl-$(C_1-C_4)$-alkylsulfonyl,
$(C_6-C_{14})$-Aryl-$(C_1-C_4)$-alkylsulfinyl, $(C_6-C_{14})$-Aryl,
$(C_6-C_{14})$-Aryloxy, $(C_3-C_{13})$-Heteroaryl und
$(C_3-C_{13})$-Heteroaryloxy

und 1 oder 2 Wasserstoffatome durch 1 oder 2 gleiche oder verschiedene Reste aus der Reihe

Carboxy, Amino, $(C_1-C_8)$-Alkylamino, Hydroxy, $(C_1-C_4)$-Alkoxy, Halogen, Di-$(C_1-C_8)$-alkylamino, Carbamoyl, Sulfamoyl, $(C_1-C_4)$-Alkoxycarbonyl, $(C_6-C_{14})$-Aryl und $(C_6-C_{14})$-Aryl-$(C_1-C_5)$-alkyl ersetzt sind;

a$_2$) $(C_6-C_{14})$-Aryl, $(C_7-C_{15})$-Aroyl, $(C_6-C_{14})$-Arylsulfonyl, $(C_3-C_{13})$-Heteroaryl, oder $(C_3-C_{13})$-Hete-

roaroyl;

$a_3$) Carbamoyl, das gegebenenfalls am Stickstoff durch ($C_1$-$C_8$)-Alkyl, ($C_6$-$C_{14}$)-Aryl oder ($C_6$-$C_{14}$)-Aryl-($C_1$-$C_5$)-alkyl substituiert sein kann;

wobei in den unter $a_1$), $a_2$) und $a_3$) definierten Resten die Aryl-, Heteroaryl-, Aroyl-, Arylsulfonyl- und Heteroaroyl-Gruppen gegebenenfalls durch 1, 2, 3 oder 4 Reste aus der Reihe
Carboxy, Amino, Nitro, ($C_1$-$C_8$)-Alkylamino, Hydroxy, ($C_1$-$C_6$)-Alkyl, ($C_1$-$C_6$)-A)koxy, ($C_6$-$C_{14}$)-Aryl, ($C_7$-$C_{15}$)-Aroyl, Halogen, Cyano, Di-($C_1$-$C_8$)-alkylamino, Carbamoyl, Sulfamoyl und ($C_1$-$C_6$)-Alkoxycarbonyl substituiert sind;

P eine direkte Bindung oder einen Rest der Formel II,

$$- NR(2) -(U)- CO - \qquad (II)$$

worin

R(2) Wasserstoff, Methyl oder eine Urethan-Schutzgruppe bedeutet,
U ($C_3$-$C_8$)-Cycloalkyliden, ($C_6$-$C_{14}$)-Aryliden, ($C_3$-$C_{13}$)-Heteroaryliden, ($C_6$-$C_{14}$)-Aryl-($C_1$-$C_6$)-alkyliden, welche gegebenenfalls substituiert sein können, oder $[CHR(3)]_n$ bedeutet,
wobei n 1 - 8, vorzugsweise 1 - 6 ist,

R(3) unabhängig voneinander Wasserstoff, ($C_1$-$C_6$)-Alkyl, ($C_3$-$C_8$)-Cycloalkyl, ($C_6$-$C_{14}$)-Aryl, ($C_3$-$C_{13}$)-Heteroaryl, bedeutet, die mit Ausnahme des Wasserstoffs jeweils gegebenenfalls durch Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pyridyl, 3-Pyridyl oder Cyclohexyl monosubstituiert sind, oder

worin R(2) und R(3) zusammen mit den diese tragenden Atomen ein mono-, bi- oder tricyclisches Ringsystem mit 2 bis 15 C-Atomen bilden;

A wie P definiert ist;

B eine basische Aminosäure in der L- oder D-Konfiguration, die in der Seitenkette substituiert sein kann;

C eine Verbindung der Formel III a oder III b

G'-G'-Gly

(III a)

$$G'-NH-(CH_2)_p-CO$$

(III b)

worin

p 2 bis 8 ist, und

G' unabhängig voneinander einen Rest der Formel IV

$$-NR(4)-CHR(5)-CO- \qquad (IV)$$

worin

R(4) und R(5) zusammen mit den diese tragenden Atomen ein heterocyclisches mono-, bi- oder tricyclisches Ringsystem mit 2 bis 15 C-Atomen bilden;

E den Rest einer neutralen, sauren oder basischen, aliphatischen oder alicyclisch-aliphatischen Aminosäure;

F den Rest einer neutralen, sauren oder basischen, aliphatischen oder aromatischen Aminosäure, die in der Seitenkette substituiert sein kann, oder eine direkte Bindung;

(D)Q D-Tic, D-Phe, D-Oic, D-Thi oder D-Nal, die gegebenenfalls durch Halogen, Methyl oder Methoxy substituiert sein können oder einen Rest der nachstehenden Formel (V)

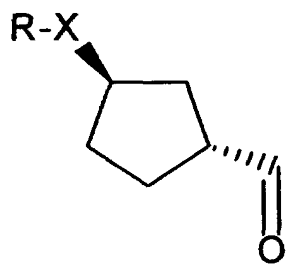

(V)

worin

X für Sauerstoff, Schwefel oder eine direkte Bindung steht;

R Wasserstoff, $(C_1-C_8)$-Alkyl, $(C_3-C_8)$-Cycloalkyl, $(C_6-C_{14})$-Aryl, $(C_6-C_{14})$-Aryl-$(C_1-C_4)$-alkyl, wobei der Alicyclus gegebenenfalls durch Halogen, Methyl oder Methoxy substituiert sein kann;

G wie G' oben definiert ist oder eine direkte Bindung;

F' wie F definiert ist, einen Rest $-NH-(CH_2)_q-$, mit q = 2 bis 8, oder, falls G keine direkte Bindung bedeutet, eine direkte Bindung;

I -OH, $-NH_2$ oder $NHC_2H_5$;

K den Rest $-NH-(CH_2)_x-CO-$ mit x = 1 - 4 oder eine direkte Bindung, und

M wie F definiert ist,

sowie deren physiologisch verträgliche Salze,
zur Herstellung von Arzneimitteln zur Behandlung von chronisch fibrogenetischen Lebererkrankungen (Leberzirrhose und Leberfibrose) und akuten Lebererkrankungen und zur Prävention des hepatorenalen Syndroms.

**2.** Verwendung nach Anspruch 1 eines Bradykinin-Antagonisten der Formel I, in welcher bedeuten

Z Wasserstoff oder wie unter $a_1$), $a_2$) oder $a_3$) definiert,

P eine Bindung oder ein Rest der Formel II

$$- NR(2) -(U)- CO - \quad (II)$$

mit U gleich CHR(3) und

R(3) wie oben definiert,
R(2) gleich H oder $CH_3$,

A eine Bindung.

**3.** Verwendung nach Anspruch 1 eines Bradykinin-Antagonisten der Formel I, in welcher bedeuten

Z Wasserstoff oder wie unter $a_1$), $a_2$) oder $a_3$) definiert,

P eine Bindung oder ein Rest der Formel II

$$- NR(2) -(U)- CO - \qquad (II)$$

mit U gleich CHR(3) und
R(3) unabhängig voneinander Wasserstoff, ($C_1$-$C_6$)-Alkyl, ($C_3$-$C_8$)-Cycloalkyl, ($C_6$-$C_{14}$)-Aryl, ($C_3$-$C_{13}$)-Heteroaryl, die mit Ausnahme des Wasserstoffs jeweils gegebenenfalls durch
Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind, oder
worin R(2) und R(3) zusammen mit den diese tragenden Atomen ein mono-, bi- oder tricyclisches Ringsystem mit 2 bis 15 C-Atomen bilden;
R(2) gleich H oder $CH_3$,

A eine Bindung;

(D)Q D-Tic.

**4.** Verwendung eines Bradykinin-Antagonisten oder deren physiologisch verträglichen Salze zur Herstellung von Arzneimitteln zur Behandlung von chronisch fibrogenetischen Lebererkrankungen (Leberzirrhose und Leberfibrose) und akuten Lebererkrankungen und zur Prävention des hepatorenalen Syndroms nach Anspruch 1, **dadurch gekennzeichnet daß** der Bradykinin-Antagonist
H-D-Arg-Arg-Pro-Hyp-Gly-Thi-Ser-D-Tic-Oic-Arg-OH (HOE 140),
para-Guanidobenzoyl-Arg-Pro-Hyp-Gly-Thi-Ser-D-Tic-Oic-Arg-OH,
H-D-Arg-Arg-Pro-Hyp-Gly-Phe-Ser-D-HypE(transpropyl)-Oic-Arg-OH,
H-D-Arg-Arg-Pro-Hyp-Gly-Cpg-Ser-D-Cpg-Cpg-Arg-OH,
H-D-Arg-Arg-Pro-Pro-Gly-Thi-Ser-D-Tic-Oic-Arg-OH,
H-Arg(Tos)-Pro-Hyp-Gly-Thi-Ser-D-Tic-Oic-Arg-OH,
H-Arg(Tos)-Pro-Hyp-Gly-Phe-Ser-D-Tic-Oic-Arg-OH,
H-D-Arg-Arg-Pro-Hyp-Gly-Phe-Ser-D-Tic-Oic-Arg-OH,
Fmoc-D-Arg-Arg-Pro-Hyp-Gly-Thi-Ser-D-Tic-Oic-Arg-OH,
Fmoc-Aoc-D-Arg-Arg-Pro-Hyp-Gly-Thi-Ser-D-Tic-Oic-Arg-OH,
Fmoc-ε-Aminocaproyl-D-Arg-Arg-Pro-Hyp-Gly-Thi-Ser-D-Tic-Oic-Arg-OH,
Benzoyl-D-Arg-Arg-Pro-Hyp-Gly-Thi-Ser-D-Tic-Oic-Arg-OH,
Cyclohexylcarbonyl-D-Arg-Arg-Pro-Hyp-Gly-Thi-Ser-D-Tic-Oic-Arg-OH,
Fmoc-Aeg(Fmoc)-D-Arg-Arg-Pro-Hyp-Gly-Thi-Ser-D-Tic-Oic-Arg-OH,
Fmoc-Aeg(Fmoc)-Arg-Pro-Hyp-Gly-Thi-Ser-D-Tic-Oic-Arg-OH,
Indol-3-yl-acetyl-D-Arg-Arg-Pro-Hyp-Gly-Thi-Ser-D-Tic-Oic-Arg-OH oder
Dibenzylacetyl-D-Arg-Arg-Pro-Hyp-Gly-Thi-Ser-D-Tic-Oic-Arg-OH ist.

**5.** Verwendung eines Bradykinin-Antagonisten gemäß Anspruch 4, **dadurch gekennzeichnet, daß** der Bradykinin-Antagonist,
H-D-Arg-Arg-Pro-Hyp-Gly-Thi-Ser-D-Tic-Oic-Arg-OH (HOE 140) oder
para-Guanidobenzoyl-Arg-Pro-Hyp-Gly-Thi-Ser-D-Tic-Oic-Arg-OH ist.

**6.** Verwendung eines Bradykinin-Antagonisten gemäß Anspruch 4, **dadurch gekennzeichnet, daß** der Bradykinin-Antagonist,

H-D-Arg-Arg-Pro-Hyp-Gly-Thi-Ser-D-Tic-Oic-Arg-OH (HOE 140) ist.

## Claims

**1.** The use of a bradykinin antagonist of the formula I

$$Z - P - A - B - C - E - F - K - (D)Q - G - M - F' - I \quad (I),$$

in which:

Z is $a_1$) hydrogen, ($C_1$-$C_8$)-alkyl, ($C_1$-$C_8$)-alkanoyl, ($C_1$-$C_8$)-alkoxycarbonyl, ($C_3$-$C_8$)-cycloalkyl, ($C_4$-$C_9$)-cycloalkanoyl or ($C_1$-$C_8$)-alkylsulfonyl,

in which 1, 2 or 3 hydrogen atoms in each case are optionally replaced by 1, 2 or 3 identical or different radicals from the group consisting of

carboxyl, NHR(1), [($C_1$-$C_4$)-alkyl]NR(1) or [($C_6$-$C_{10}$)-aryl-($C_1$-$C_4$)-alkyl]NR(1), where R(1) is hydrogen or a urethane protective group, ($C_1$-$C_4$)-alkyl, ($C_1$-$C_8$)-alkylamino, ($C_6$-$C_{10}$)-aryl-($C_1$-$C_4$)-alkylamino, hydroxyl, ($C_1$-$C_4$)-alkoxy, halogen, di-($C_1$-$C_8$)-alkylamino, di-[($C_6$-$C_{10}$)-aryl-($C_1$-$C_4$)-alkyl]-amino, carbamoyl, phthalimido, 1,8-naphthalimido, sulfamoyl, ($C_1$-$C_4$)-alkoxycarbonyl, ($C_6$-$C_{14}$)-aryl and ($C_6$-$C_{14}$)-aryl-($C_1$-$C_5$)-alkyl,

or in which 1 hydrogen atom in each case is optionally replaced by a radical from the group consisting of

($C_3$-$C_8$)-cycloalkyl, ($C_1$-$C_6$)-alkylsulfonyl,
($C_1$-$C_6$)-alkylsulfinyl,
($C_6$-$C_{14}$)-aryl-($C_1$-$C_4$)-alkylsulfonyl,
($C_6$-$C_{14}$)-aryl-($C_1$-$C_4$)-alkylsulfinyl, ($C_6$-$C_{14}$)-aryl,
($C_6$-$C_{14}$)-aryloxy, ($C_3$-$C_{13}$)-heteroaryl and
($C_3$-$C_{13}$)-heteroaryloxy

and 1 or 2 hydrogen atoms are replaced by 1 or 2 identical or different radicals from the group consisting of

carboxyl, amino, ($C_1$-$C_8$)-alkylamino, hydroxyl, ($C_1$-$C_4$)-alkoxy, halogen, di-($C_1$-$C_8$)-alkylamino, carbamoyl, sulfamoyl, ($C_1$-$C_4$)-alkoxycarbonyl, ($C_6$-$C_{14}$)-aryl and ($C_6$-$C_{14}$)-aryl-($C_1$-$C_5$)-alkyl;

$a_2$) ($C_6$-$C_{14}$)-aryl, ($C_7$-$C_{15}$)-aroyl, ($C_6$-$C_{14}$)-arylsulfonyl, ($C_3$-$C_{13}$)-heteroaryl or ($C_3$-$C_{13}$)-heteroaroyl;

$a_3$) carbamoyl which can optionally be substituted on the nitrogen by ($C_1$-$C_8$)-alkyl, ($C_6$-$C_{14}$)-aryl or ($C_6$-$C_{14}$)-aryl-($C_1$-$C_5$)-alkyl;

where in the radicals defined under $a_1$), $a_2$) and $a_3$) the aryl, heteroaryl, aroyl, arylsulfonyl and heteroaroyl groups are optionally substituted by 1, 2, 3 or 4 radicals from the group consisting of

carboxyl, amino, nitro, ($C_1$-$C_8$)-alkylamino, hydroxyl, ($C_1$-$C_6$)-alkyl, ($C_1$-$C_6$)-alkoxy, ($C_6$-$C_{14}$)-aryl, ($C_7$-$C_{15}$)-aroyl, halogen, cyano, di-($C_1$-$C_8$)-alkylamino, carbamoyl, sulfamoyl and ($C_1$-$C_6$)-alkoxycarbonyl;

P is a direct bond or a radical of the formula II,

$$- NR(2) -(U)- CO - \quad (II)$$

in which

R(2) is hydrogen, methyl or a urethane protective group,

U is ($C_3$-$C_8$)-cycloalkylidene, ($C_6$-$C_{14}$)-arylidene, ($C_3$-$C_{13}$)-heteroarylidene, ($C_6$-$C_{14}$)-aryl-($C_1$-$C_6$)-alkylidene, which can optionally be substituted, or $[CHR(3)]_n$ ,

where n is 1 - 8, preferably 1 - 6,

R(3) independently of one another is hydrogen, ($C_1$-$C_6$)-alkyl, ($C_3$-$C_8$)-cycloalkyl, ($C_6$-$C_{14}$)-aryl, ($C_3$-$C_{13}$)-heteroaryl, which with the exception of hydrogen are each optionally monosubstituted by amino, substituted amino, amidino, substituted amidino, hydroxyl, carboxyl, carbamoyl, guanidino, substituted guanidino, ureido, substituted ureido, mercapto, methylmercapto, phenyl, 4-chlorophenyl, 4-fluorophenyl, 4-nitrophenyl, 4-methoxyphenyl, 4-hydroxyphenyl, phthalimido, 1,8-naphthalimido, 4-imidazolyl, 3-indolyl, 2-thienyl, 3-thienyl, 2-pyridyl, 3-pyridyl or cyclohexyl, or

in which R(2) and R(3), together with the atoms carrying them, form a mono-, bi- or tricyclic ring system having 2 to 15 carbon atoms;

A is defined as P;

B is a basic amino acid in the L- or D-configuration, which can be substituted in the side chain;

C is a compound of the formula III a or III b

G'-G'-Gly

(III a)

$$G'-NH-(CH_2)_p-CO$$

(III b)

in which

p is 2 to 8, and

G' independently of one another is a radical of the formula IV

-NR(4)-CHR(5)-CO- (IV)

in which
R(4) and R(5), together with the atoms carrying them, form a heterocyclic mono-, bi- or tricyclic ring system having 2 to 15 carbon atoms;

E is the radical of a neutral, acidic or basic, aliphatic or alicyclicaliphatic amino acid;

F is the radical of a neutral, acidic or basic, aliphatic or aromatic amino acid which can be substituted in the side chain, or a direct bond;

(D)Q is D-Tic, D-Phe, D-Oic, D-Thi or D-Nal, which can optionally be substituted by halogen, methyl or methoxy or a radical of the formula (V) below

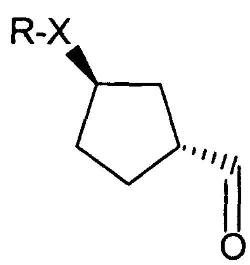

(V)

in which

X is oxygen, sulfur or a direct bond;

R is hydrogen, $(C_1-C_8)$-alkyl, $(C_3-C_8)$-cycloalkyl, $(C_6-C_{14})$-aryl, $(C_6-C_{14})$-aryl-$(C_1-C_4)$-alkyl, where the alicyclic system can optionally be substituted by halogen, methyl or methoxy;

G is defined as G' above or is a direct bond;

F' is defined as F, is a radical $-NH-(CH_2)_q-$, where q = 2 to 8, or, if G is not a direct bond, is a direct bond;

I -OH, $-NH_2$ or $NHC_2H_5$;

K is the radical $-NH-(CH_2)_x-CO-$ where x = 1 - 4 or is a direct bond, and

M is defined as F,

and its physiologically tolerable salts.
for the production of pharmaceuticals for the treatment of chronic fibrogenetic liver disorders (hepatic cirrhosis and hepatic fibrosis) and acute liver disorders and for the prevention of hepatorenal syndrome.

**2.** The use as claimed in claim 1 of a bradykinin antagonist of the formula I in which:

Z is hydrogen or as defined under $a_1$), $a_2$) or $a_3$),
P is a bond or a radical of the formula II

$$- NR(2) -(U)- CO - \quad (II)$$

where U is CHR(3) and
R(3) is as defined above,
R(2) is H or $CH_3$,
A is a bond.

**3.** The use as claimed in claim 1 of a bradykinin antagonist of the formula I, in which:

Z is hydrogen or as defined under $a_1$), $a_2$) or $a_3$),

P is a bond or a radical of the formula II

$$- NR(2) -(U)- CO - \quad (II)$$

where U is CHR(3) and
R(3) independently of one another is hydrogen, $(C_1-C_6)$-alkyl, $(C_3-C_8)$-cycloalkyl, $(C_6-C_{14})$-aryl, $(C_3-C_{13})$-heteroaryl, which with the exception of the hydrogen are each optionally monosubstituted by amino, substituted amino, hydroxyl, carboxyl, carbamoyl, guanidino, substituted guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pyridyl or cyclohexyl, or
in which R(2) and R(3), together with the atoms carrying them, form a mono-, bi- or tricyclic ring system having 2 to 15 carbon atoms,
R(2) is H or $CH_3$;

A is a bond;

(D)Q is D-Tic.

4. The use of a bradykinin antagonist or its physiologically tolerable salts for the production of medicaments for the treatment of chronic fibrogenetic liver disorders (hepatic cirrhosis and hepatic fibrosis) and acute liver disorders and for the prevention of hepatorenal syndrome as claimed in claim 1, wherein the bradykinin antagonist is
H-D-Arg-Arg-Pro-Hyp-Gly-Thi-Ser-D-Tic-Oic-Arg-OH (HOE 140),
para-guanidobenzoyl-Arg-Pro-Hyp-Gly-Thi-Ser-D-Tic-Oic-Arg-OH,
H-D-Arg-Arg-Pro-Hyp-Gly-Phe-Ser-D-HypE(transpropyl)-Oic-Arg-OH,
H-D-Arg-Arg-Pro-Hyp-Gly-Cpg-Ser-D-Cpg-Cpg-Arg-OH,
H-D-Arg-Arg-Pro-Pro-Gly-Thi-Ser-D-Tic-Oic-Arg-OH,
H-Arg(Tos)-Pro-Hyp-Gly-Thi-Ser-D-Tic-Oic-Arg-OH,
H-Arg(Tos)-Pro-Hyp-Gly-Phe-Ser-D-Tic-Oic-Arg-OH,
H-D-Arg-Arg-Pro-Hyp-Gly-Phe-Ser-D-Tic-Oic-Arg-OH,
Fmoc-D-Arg-Arg-Pro-Hyp-Gly-Thi-Ser-D-Tic-Oic-Arg-OH,
Fmoc-Aoc-D-Arg-Arg-Pro-Hyp-Gly-Thi-Ser-D-Tic-Oic-Arg-OH,
Fmoc-ε-aminocaproyl-D-Arg-Arg-Pro-Hyp-Gly-Thi-Ser-D-Tic-Oic-Arg-OH,
benzoyl-D-Arg-Arg-Pro-Hyp-Gly-Thi-Ser-D-Tic-Oic-Arg-OH,
cyclohexylcarbonyl-D-Arg-Arg-Pro-Hyp-Gly-Thi-Ser-D-Tic-Oic-Arg-OH,
Fmoc-Aeg(Fmoc)-D-Arg-Arg-Pro-Hyp-Gly-Thi-Ser-D-Tic-Oic-Arg-OH,
Fmoc-Aeg(Fmoc)-Arg-Pro-Hyp-Gly-Thi-Ser-D-Tic-Oic-Arg-OH,
indol-3-yl-acetyl-D-Arg-Arg-Pro-Hyp-Gly-Thi-Ser-D-Tic-Oic-Arg-OH or
dibenzylacetyl-D-Arg-Arg-Pro-Hyp-Gly-Thi-Ser-D-Tic-Oic-Arg-OH.

5. The use of a bradykinin antagonist as claimed in claim 4, wherein the bradykinin antagonist is
H-D-Arg-Arg-Pro-Hyp-Gly-Thi-Ser-D-Tic-Oic-Arg-OH (HOE 140) or
para-guanidobenzoyl-Arg-Pro-Hyp-Gly-Thi-Ser-D-Tic-Oic-Arg-OH.

6. The use of a bradykinin antagonist as claimed in claim 4, wherein the bradykinin antagonist is
H-D-Arg-Arg-Pro-Hyp-Gly-Thi-Ser-D-Tic-Oic-Arg-OH (HOE 140).

## Revendications

1. Utilisation d'un antagoniste de bradykinine de formule I

$$Z\text{-}P\text{-}A\text{-}B\text{-}C\text{-}E\text{-}F\text{-}K\text{-}(D)Q\text{-}G\text{-}M\text{-}F'\text{-}I \qquad (I)$$

dans laquelle :

Z représente

$a_1$) un atome d'hydrogène, un groupe alkyle en $C_1$-$C_8$, alcanoyle en $C_1$-$C_8$, alcoxy($C_1$-$C_8$)-carbonyle, cycloalkyle en $C_3$-$C_8$, cycloalcanoyle en $C_4$-$C_9$ ou alkyl($C_1$-$C_8$)sulfonyle, dans chacun desquels 1, 2 ou 3 atomes d'hydrogène sont éventuellement remplacés par 1, 2 ou 3 radicaux, identiques ou différents, choisis dans la série des groupes
carboxy, NHR(1), [alkyl($C_1$-$C_4$)]NR(1) et [aryl($C_6$-$C_{10}$)-alkyl($C_1$-$C_4$)]NR(1),
R(1) représentant un atome d'hydrogène ou un groupe protecteur uréthanne, des groupes alkyle en $C_1$-$C_4$, alkyl ($C_1$-$C_8$) amino, aryl-($C_6$-$C_{10}$)-alkyl($C_1$-$C_4$)amino, hydroxy, alcoxy en $C_1$-$C_4$, des atomes d'halogène, des groupes dialkyl ($C_1$-$C_8$) amino, di[aryl-($C_6$-$C_{10}$)-alkyl($C_1$-$C_4$)]amino, carbamoyle, phtalimido, 1,8-naphtalimido, sulfamoyle, alcoxy($C_1$-$C_4$)carbonyle, aryle en $C_6$-$C_{14}$ et aryl ($C_6$-$C_{14}$) -alkyl ($C_1$-$C_5$), ou dans chacun desquels un atome d'hydrogène est éventuellement remplacé par un radical choisi dans la série des groupes cycloalkyle en $C_3$-$C_8$, alkyl ($C_1$-$C_6$)-sulfonyle, alkyl ($C_1$-$C_6$)sulfinyle, aryl-($C_6$-$C_{14}$)-alkyl ($C_1$-$C_4$)sulfonyle, aryl-($C_6$-$C_{14}$)-alkyl($C_1$-$C_4$)sulfinyle, aryle en $C_6$-$C_{14}$, aryloxy en $C_6$-$C_{14}$, hétéroaryle en $C_3$-$C_{13}$ et hétéroaryloxy en $C_3$-$C_{13}$,
et 1 ou 2 atomes d'hydrogène sont remplacés par 1 ou 2 radicaux, identiques ou différents, choisis dans la série des groupes carboxy, amino, alkyl ($C_1$-$C_8$)amino, hydroxy, alcoxy en $C_1$-$C_4$, des atomes d'halogène, des groupes dialkyl ($C_1$-$C_8$)amino, carbamoyle, sulfamoyle, alcoxy($C_1$-$C_4$)carbonyle, aryle en $C_6$-$C_{14}$ et aryl($C_6$-$C_{14}$)-alkyle($C_1$-$C_5$) ;

a2) un groupe aryle en $C_6$-$C_{14}$, aroyle en $C_7$-$C_{15}$, aryl($C_6$-$C_{14}$)sulfonyle, hétéroaryle en $C_3$-$C_{13}$ ou hétéroaroyle en $C_3$-$C_{13}$ ;

a3) un groupe carbamoyle qui peut éventuellement être substitué sur l'atome d'azote par alkyle en $C_1$-$C_8$, aryle en $C_6$-$C_{14}$ ou aryl($C_6$-$C_{14}$)-alkyle($C_1$-$C_5$) ;
dans les radicaux définis en $a_1$), $a_2$) et $a_3$), les groupes aryle, hétéroaryle, aroyle, arylsulfonyle et hétéroaroyle étant éventuellement substitués par 1, 2, 3 ou 4 radicaux choisis dans la série des groupes carboxy, amino, nitro, alkyl($C_1$-$C_8$)amino, hydroxy, alkyle en $C_1$-$C_6$, alcoxy en $C_1$-$C_6$, aryle en $C_6$-$C_{14}$, aroyle en $C_7$-$C_{15}$, des atomes d'halogène, des groupes cyano, dialkyl($C_1$-$C_8$)-amino, carbamoyle, sulfamoyle et alcoxy($C_1$-$C_6$)carbonyle ;

P représente une liaison directe ou un radical de formule II,

$$-NR(2)-(U)-CO- \quad (II)$$

dans laquelle

R(2) représente un atome d'hydrogène, le groupe méthyle ou un groupe protecteur uréthanne,

U représente un groupe cycloalkylidène en $C_3$-$C_8$, arylidène en $C_6$-$C_{14}$, hétéroarylidène en $C_3$-$C_{13}$, aryl ($C_6$-$C_{14}$)-alkylidène($C_1$-$C_6$),
qui peuvent éventuellement être substitués, ou $[CHR(3)]_n$,
n allant de 1 à 8, de préférence de 1 à 6,
R(3) représentant, indépendamment les uns des autres, un atome d'hydrogène, un groupe alkyle en $C_1$-$C_6$, cycloalkyle en $C_3$-$C_8$, aryle en $C_6$-$C_{14}$, hétéroaryle en $C_3$-$C_{13}$, qui, à l'exception de l'atome d'hydrogène, sont éventuellement monosubstitués chacun par un groupe amino, amino substitué, amidino, amidino substitué, hydroxy, carboxy, carbamoyle, guanidino, guanidino substitué, urêido, uréido substitué, mercapto, méthylmercapto, phényle, 4-chlorophényle, 4-fluorophényle, 4-nitrophényle, 4-méthoxyphényle, 4-hydroxyphényle, phtalimido, 1,8-naphtalimido, 4-imidazolyle, 3-indolyle, 2-thiényle, 3-thiényle, 2-pyridyle, 3-pyridyle ou cyclohexyle, ou
dans laquelle R(2) et R(3) forment ensemble, avec les atomes qui les portent, un système cyclique mono-, bi- ou tricyclique ayant de 2 à 15 atomes de carbone ;

A est défini comme P ;

B représente un aminoacide basique en configuration L ou D, qui peut être substitué sur la chaîne latérale ;

C représente un composé de formule III a ou III b

G'-G'-Gly

(III a)

$$G'-NH-(CH_2)_p-CO$$

(III b)

formules dans lesquelles

p va de 2 à 8 et

G', indépendamment les uns des autres, représente un radical de formule IV

$$-NR(4)-CHR(5)-CO- \quad (IV)$$

dans laquelle
R(4) et R(5) forment ensemble, avec les atomes qui les portent, un système cyclique mono-, bi- ou tricyclique hétérocyclique ayant de 2 à 15 atomes de carbone ;

E représente le reste d'un aminoacide neutre, acide ou basique, aliphatique ou alicyclique-aliphatique ;

F représente le reste d'un aminoacide aliphatique ou aromatique, neutre, acide ou basique, qui peut être substitué sur la chaîne latérale, ou une liaison directe ;

(D)Q représente D-Tic, D-Phe, D-Oic, D-Thi ou D-Nal, qui peuvent éventuellement être substitués par halogène, méthyle ou méthoxy, ou un radical de formule (V) ci-après

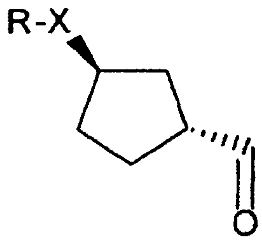

(V)

dans laquelle

X représente un atome d'oxygène, de soufre ou une liaison directe ;

R représente un atome d'hydrogène, un groupe alkyle en $C_1$-$C_8$, cycloalkyle en $C_3$-$C_8$, aryle en $C_6$-$C_{14}$, aryl ($C_6$-$C_{14}$,) -alkyle ($C_1$-$C_4$), le système alicyclique pouvant éventuellement être substitué par halogène, méthyle ou méthoxy ;

G est défini comme G' ci-dessus ou est une liaison directe ;

F' est défini comme F, représente un radical -NH-$(CH_2)_q$-, où q = 2 à 8, ou représente une liaison directe lorsque G ne représente pas une liaison directe ;

I représente -OH, $-NH_2$ ou $-NHC_2H_5$ ;

K représente le radical -NH-$(CH_2)_x$-CO- où x = 1 à 4 ou une liaison directe ; et

M est défini comme F,

ainsi que leurs sels physiologiquement acceptables, pour la fabrication de médicaments destinés au traitement d'hépatopathies fibrogénétiques chroniques (cirrhose et fibrose hépatique) et d'hépatopathies aiguës, et à la prévention du syndrome hépatorénal.

**2.** Utilisation selon la revendication 1 d'un antagoniste de bradykinine de formule I, dans laquelle

Z représente un atome d'hydrogène ou est défini comme en $a_1$), $a_2$) ou $a_3$),
P représente une liaison ou un radical de formule II

$$-NR(2)-(U)-CO- \qquad (II)$$

où U représente CHR(3) et
R(3) est tel que défini plus haut,
R(2) représente H ou $CH_3$,
A représente une liaison.

**3.** Utilisation selon la revendication 1 d'un antagoniste de bradykinine de formule I, dans laquelle

Z représente un atome d'hydrogène ou est défini comme en $a_1$), $a_2$) ou $a_3$),

P représente une liaison ou un radical de formule II

-NR(2)-(U)-CO- (II)

dans laquelle
U représente CHR(3) et
R(3) représente, indépendamment les uns des autres, un atome d'hydrogène, un groupe alkyle en $C_1$-$C_6$, cycloalkyle en $C_3$-$C_8$, aryle en $C_6$-$C_{14}$, hétéroaryle en $C_3$-$C_{13}$ qui, à l'exception de l'atome d'hydrogène, sont éventuellement monosubstitués chacun par
un groupe amino, amino substitué, hydroxy, carboxy, carbamoyle, guanidino, guanidino substitué, uréido, mercapto, méthylmercapto, phényle, 4-chlorophényle, 4-fluorophényle, 4-nitrophényle, 4-méthoxyphényle, 4-hydroxyphényle, phtalimido, 4-imidazolyle, 3-indolyle, 2-thiényle, 3-thiényle, 2-pyridyle, 3-pyridyle ou cyclohexyle, ou
dans laquelle R(2) et R(3) forment ensemble, avec les atomes qui les portent, un système cyclique mono-, bi- ou tricyclique ayant de 2 à 15 atomes de carbone ; R(2) représente H ou $CH_3$,

A représente une liaison ;

D(Q) représente D-Tic.

**4.** Utilisation d'un antagoniste de bradykinine ou de ses sels physiologiquement acceptables, pour la fabrication de médicaments destinés au traitement d'hépatopathies fibrogénétiques chroniques (cirrhose et fibrose hépatique) et d'hépatopathies aiguës et à la prévention du syndrome hépatorénal selon la revendication 1, **caractérisée en ce que** l'antagoniste de bradykinine est
H-D-Arg-Arg-Pro-Hyp-Gly-Thi-Ser-D-Tic-Oic-Arg-OH (HOE 140),
para-guanidobenzoyl-Arg-Pro-Hyp-Gly-Thi-Ser-D-Tic-Oic-Arg-OH,
H-D-Arg-Arg-Pro-Hyp-Gly-Phe-Ser-D-HypE(trans-propyl)-Oic-Arg-OH,
H-D-Arg-Arg-Pro-Hyp-Gly-Cpg-Ser-D-Cpg-Cpg-Arg-OH,
H-D-Arg-Arg-Pro-Pro-Gly-Thi-Ser-D-Tic-Oic-Arg-OH,
H-Arg(Tos)-Pro-Hyp-Gly-Thi-Ser-D-Tic-Oic-Arg-OH,
H-Arg(Tos)-Pro-Hyp-Gly-Phe-Ser-D-Tic-Oic-Arg-OH,
H-D-Arg-Arg-Pro-Hyp-Gly-Phe-Ser-D-Tic-Oic-Arg-OH,
Fmoc-D-Arg-Arg-Pro-Hyp-Gly-Thi-Ser-D-Tic-Oic-Arg-OH,
Fmoc-Aoc-D-Arg-Arg-Pro-Hyp-Gly-Thi-Ser-D-Tic-Oic-Arg-OH,
Fmoc-ε-aminocaproyl-D-Arg-Arg-Pro-Hyp-Gly-Thi-Ser-D-Tic-Oic-Arg-OH,
benzoyl-D-Arg-Arg-Pro-Hyp-Gly-Thi-Ser-D-Tic-Oic-Arg-OH,
cyclohexylcarbonyl-D-Arg-Arg-Pro-Hyp-Gly-Thi-Ser-D-Tic-Oic-Arg-OH,
Fmoc-Aeg(Fmoc)-D-Arg-Arg-Pro-Hyp-Gly-Thi-Ser-D-Tic-Oic-Arg-OH,
Fmoc-Aeg(Fmoc)-Arg-Pro-Hyp-Gly-Thi-Ser-D-Tic-Oic-Arg-OH,
Indol-3-yl-acétyl-D-Arg-Arg-Pro-Hyp-Gly-Thi-Ser-D-Tic-Oic-Arg-OH ou
dibenzylacétyl-D-Arg-Arg-Pro-Hyp-Gly-Thi-Ser-D-Tic-Oic-Arg-OH.

**5.** Utilisation d'un antagoniste de bradykinine selon la revendication 4, **caractérisée en ce que** l'antagoniste de bradykinine est
H-D-Arg-Arg-Pro-Hyp-Gly-Thi-Ser-D-Tic-Oic-Arg-OH (HOE 140) ou
para-guanidobenzoyl-Arg-Pro-Hyp-Gly-Thi-Ser-D-Tic-Oic-Arg-OH.

**6.** Utilisation d'un antagoniste de bradykinine selon la revendication 4, **caractérisée en ce que** l'antagoniste de bradykinine est
H-D-Arg-Arg-Pro-Hyp-Gly-Thi-Ser-D-Tic-Oic-Arg-OH (HOE 140).